# EUROPÄISCHE PATENTANMELDUNG

(11) **EP 3 150 279 A1**
(43) Veröffentlichungstag der Anmeldung: **05.04.2017**
(21) Anmeldenummer: 16191896.6
(22) Anmeldetag: 30.09.2016
(51) Int. Cl.: B01L 3/00, C12M 1/12, C12M 1/34, G01N 15/06, G01N 27/02, G01N 27/30, G01N 33/487

(54) **VORRICHTUNG ZUM ANALYSIEREN VON BIOLOGISCHEN SUBSTANZEN IN EINER TESTLÖSUNG UND HERSTELLUNGSVERFAHREN**

(30) Priorität: 01.10.2015 DE 102015219023
(71) Anmelder: Technische Universität München, 80333 München (DE)
(72) Erfinder: LUGLI, Paolo, 85933 Hallbergmoos (DE); BHATT, Vijay Deep, 81373 München (DE); MELZER, Katharina, 82272 Moorenweis (DE)
(74) Vertreter: Isarpatent

(57) **Zusammenfassung**

Die Erfindung betrifft eine Vorrichtung (100; 200; 300; 400) zum Analysieren von biologischen Substanzen in einer Testlösung, mit einem zumindest teilweise transparenten Testsubstrat (101; 203; 303; 401) mit einem Testbereich (107a, 108a, 109a, 110a; 211; 411) zum Aufnehmen der Testlösung, einer Vielzahl von auf dem Testsubstrat (101; 203; 303; 401) angeordneten und sich in den Testbereich (107a, 108a, 109a, 110a; 211; 411) erstreckenden Elektroden (111, 106; 201, 202; 301, 302; 402, 403), wobei jeweils zumindest ein Teilbereich der Elektroden (111, 106; 201, 202; 301, 302; 402, 403) aus einem transparenten Material ausgebildet sind.

## Beschreibung

Die vorliegende Erfindung betrifft eine Vorrichtung zum Analysieren von biologischen Substanzen in einer Testlösung und ein Herstellungsverfahren für eine Vorrichtung zum Analysieren einer Testlösung.

Effiziente und präzise Methoden zur Untersuchung von Zellen sind in einer Vielzahl von Anwendungsgebieten der Biologie, Medizin oder Pharmazie von höchster Bedeutung. Derartige Methoden umfassen insbesondere die Identifizierung von bestimmten Zellbestandteilen, beispielsweise von DNA, Proteinen oder Enzymen, oder auch die Untersuchung der Veränderlichkeit dieser Zellbestandteile, um Rückschlüsse auf die Vitalität von Zellen oder auf das Wachstum von Zellen zu ziehen. Derartige Analysen werden etwa für die Untersuchung der Lebensmittel- oder Wasserqualität, für Medikamententests und Toxizitätsstudien eingesetzt.

Konventionelle Methoden zur Zelluntersuchung, wie sie auch zur Entwicklung von in-vitro Zellmodellen für toxikologische Untersuchungen und Drogen- und Medikamententests verwendet werden, sind hierbei optische Untersuchungsverfahren. Hierzu werden üblicherweise Teile der Zelle mit fluoreszierenden Molekülen, Chromophoren, fluoreszierenden Farbstoffen oder radioaktiven Molekülen markiert, um diese Zellbestandteile besser untersuchen zu können. Dadurch können jedoch bestimmte chemische oder biologische Eigenschaften der zu untersuchenden Zellbestandteile verändert werden, wodurch eine präzise Untersuchung erschwert wird.

Alternativen zur Überprüfung von fluidischen Mikrosystemen bieten elektrische Verfahren, insbesondere die Impedanzspektroskopie. So ist aus der DE 102 34 487 A1 ein Impedanzmessverfahren für in einer Flüssigkeit suspendierte Partikel bekannt. Zwischen Elektroden wird hierbei ein elektrisches Feld aufgebaut und durch Messen der Impedanzänderung der Elektroden lassen sich Rückschlüsse auf sich bewegende Partikel zwischen den Elektroden ziehen, da diese mit dem elektrischen Feld wechselwirken.

Die Elektroden bestehen typischerweise aus Metallen wie etwa Gold, was dazu führt, dass die Elektroden optisch intransparent sind. Dadurch ist es nicht möglich, gleichzeitig optische Verfahren anzuwenden.

Die vorliegende Erfindung schafft eine Vorrichtung zum Analysieren von biologischen Substanzen in einer Testlösung nach Anspruch 1.

Demgemäß schafft die vorliegende Erfindung eine Vorrichtung zum Analysieren von biologischen Substanzen in einer Testlösung, mit einem zumindest teilweise transparenten Testsubstrat mit einem Testbereich zum Aufnehmen der Testlösung. Weiter umfasst die Vorrichtung eine Vielzahl von auf dem Testsubstrat angeordneten und sich in den Testbereich erstreckenden Elektrode, wobei jeweils zumindest ein Teilbereich der Elektroden im Testbereich aus einem leitfähigen und transparenten Material ausgebildet ist.

Unter "Testlösung" ist insbesondere ein Mikrofluid zu verstehen. Die Testlösung kann mehrkomponentige Substanzen enthalten, beispielsweise kann die Testlösung Blut sein.

Der Begriff "Material" bezeichnet hierbei sowohl ein einkomponentiges als auch ein mehrkomponentiges Material. Insbesondere können die Elektroden verschiedene Teilbereiche umfassen, welche aus unterschiedlichen Komponenten gefertigt sind.

"Transparenz" bedeutet hierbei, dass eine Transmission von mehr als 50 %, insbesondere mehr als 70 %, vorzugsweise mehr als 90 % in einem entsprechenden Wellenlängenbereich der elektromagnetischen Strahlung vorliegt. Der entsprechende Wellenlängenbereich kann hierbei insbesondere sichtbares Licht umfassen. Insbesondere liegt der Wellenlängenbereich der elektromagnetischen Strahlung in einem sichtbaren Spektralbereich zwischen 350 nm und 800 nm. Der Wellenlängenbereich kann sich jedoch auch in den Ultraviolettbereich und/oder Infrarotbereich erstrecken.

Die Elektroden bestehen zumindest teilweise aus einem leitfähigen Material.

Darüber hinaus schafft die vorliegende Erfindung ein Herstellungsverfahren für eine Vorrichtung zum Analysieren von biologischen Substanzen in einer Testlösung mit den Merkmalen des Patentanspruchs 11.

Die Erfindung schafft demnach ein Herstellungsverfahren für eine Vorrichtung zum Analysieren von biologischen Substanzen in einer Testlösung mit den Schritten: Ausbilden eines zumindest teilweise transparenten, vorzugsweise vollständig transparenten, Testsubstrats mit einem Testbereich zum Aufnehmen der Testlösung, und Anordnen von einer Vielzahl von Elektroden auf dem Testsubstrat. Hierbei wird jeweils zumindest ein Teilbereich der Elektroden im Testbereich aus einem transparenten Material ausgebildet.

Die erfindungsgemäße Vorrichtung hat den Vorteil, dass die Elektroden zumindest abschnittsweise transparent sind, wodurch sich die Vorrichtung sowohl zum Analysieren von biologischen Substanzen in der Testlösung mittels Impedanzspektroskopie als auch zur Analyse mit Hilfe von optischen Verfahren eignet. Durch Impedanzspektroskopie können die biologischen Substanzen präzise untersucht werden, beispielsweise kann erkannt werden, ob sich Zellen oder Zellbestandteile an bestimmten Positionen der Vorrichtung in dem Testbereich befinden. Weiter können Bewegungen oder das Wachstum von einzelnen Zellen, Neuronen, einer Zellschicht oder Zellbestandteilen gemessen werden. Elektrische Analyse, insbesondere Impedanzspektroskopie hat den Vorteil einer kompakten Ausführung. Insbesondere kann die Elektronik direkt in die Vorrichtung integriert werden. Die elektronische Auswertung bietet hierbei ein hochsensitives Verfahren zur Ergänzung von optischen Verfahren. Da ein Labelling der biologischen Substanzen entfällt, besteht keine Gefahr der Verfälschung der Testergebnisse durch Änderung des Zellverhaltens.

Andererseits kann etwa durch Eingeben der Vorrichtung in ein konventionelles Mikroskop ein zusätzliches optisches Read-out ermöglicht werden. Durch Aufnehmen von Bildern oder Videos können die dadurch erhaltenen optischen Daten mit den durch Impedanzspektroskopie erhaltenen Daten korreliert werden und gemeinsam ausgewertet werden. Dadurch ist es möglich, die Genauigkeit der Analysen zu erhöhen. Durch eine parallele optische als auch elektrische Analyse der Testlösung und der z.B. darin enthaltenen Zellen und/oder Zellbestandteilen können neue elektrische Standardtests entwickelt werden und gleichzeitig mit konventionellen optischen Standardtest korreliert und damit validiert werden. Dadurch lassen sich die neuen elektrischen Tests als Standardtests in unterschiedlichen Bereichen der Biologie, Pharmazie oder Medizin etablieren. Zusätzlich zur klassischen Impedanzspektroskopie können mit dieser Vorrichtung auch neue Arten von elektrischen Messmethoden zur beispielsweise Bestimmung von Zellbewegung realisiert werden. Die Vorrichtung ist hierbei für vielfältige Anwendungen in der Biologie, Pharmazie oder Medizin geeignet.

Gemäß einer Weiterbildung der erfindungsgemäßen Vorrichtung weist das Testsubstrat eine Kavität zum Aufnehmen der Testlösung auf. Die Vorrichtung kann insbesondere Fluidkammern und/oder ein Kanalsystem und/oder Vertiefungen zum Aufnehmen der Testlösung umfassen. Durch Verwenden von Kanälen mit Zu- und Ablauf kann ein einfacher Fluidaustausch bzw. Zellaustausch ermöglicht werden. Besonders ein Mischen unterschiedlicher Substanzen bzw. Testlösungen ist durch beispielsweise eine Aufbringung einer speziellen Mikrofluidik möglich. Das erlaubt speziell auch die Analyse mehrkomponentiger Testlösungen.

Gemäß einer Weiterbildung der erfindungsgemäßen Vorrichtung umfasst das transparente Material aus welchem jeweils zumindest der Teilbereich der Elektroden ausgebildet ist, Kohlenstoff-Nanoröhren (CNTs) und/oder leitenden Oxide (z.B. ZnO) und/oder Polymere und/oder metallische Nanodrähte, insbesondere Silber-Nanodrähte, und/oder PEDOT-PSS und/oder Graphen und/oder Graphenoxid. Durch Verwendung eines geeigneten derartigen Nanomaterials können die elektrischen Eigenschaften der Elektroden optimiert werden. Beispielsweise weisen Silber-Nanodrähte eine gute Leitfähigkeit bei gleichzeitiger optischer Transparenz auf.

Gemäß einer Weiterbildung der erfindungsgemäßen Vorrichtung bestehen das Testsubstrat und die Elektroden zumindest teilweise aus einem biokompatiblen, insbesondere bioinerten Material. Ein derartiges bioinertes Material umfasst beispielsweise Poly-merstrukturen oder Kohlenstoff-Nanoröhren.

Ein "bioinertes Material" bezeichnet hierbei ein möglicherweise mehrkomponentiges Material, wobei die chemischen und/oder biologischen Wechselwirkungen zwischen dem Material bei Berührung mit biologischen Substanzen mit dieser ausbleibt. Es werden keine Mengen an toxischen Substanzen freigesetzt, so dass das bioinerte Material die biologischen Substanzen nicht merklich verändert oder gar zerstört. Bei einem biokompatiblen Material sind die Wechselwirkungen lediglich schwach und es werden nur geringe Mengen an toxischem Material abgegeben.

Gemäß einer Weiterbildung der erfindungsgemäßen Vorrichtung weisen die Elektroden jeweils einen Endbereich und einen restlichen Bereich auf. Der jeweilige Endbereich der Elektroden besteht zumindest teilweise, vorzugsweise vollständig aus einem biokompatiblen, insbesondere bioinerten Material und der jeweils restliche Bereich der Elektroden besteht aus einem Material, welches eine höhere elektrische Leitfähigkeit als die des biokompatiblen Materials aufweist. Durch eine derartige Hybridstruktur der Elektroden sind diese vorzugsweise zum größten Teil aus einem gut elektrisch leitfähigen Material, beispielsweise aus Silber-Nanodrähten ausgebildet. Der Endbereich der Elektroden besteht aus biokompatiblen, insbesondere bioinertem Material, beispielsweise aus Kohlenstoff-Nanoröhren. Die jeweiligen Endbereiche der Elektroden erstrecken sich hierbei vorzugsweise in den Testbereich des Testsubstrats, so dass in dem Testbereich, welcher zum Aufnehmen des biologischen Materials dient, die Elektroden aus einem biokompatiblen Material bestehen. Dadurch werden die biologischen Substanzen der zu untersuchenden Zellen nicht oder kaum durch die Elektroden verändert oder beeinflusst. Die derartige Vorrichtung mit Hybridstruktur der Elektroden kombiniert somit Transparenz, gute Leitfähigkeit und Biokompatibilität.

Gemäß einer Weiterbildung der erfindungsgemäßen Vorrichtung bestehen die Elektroden zumindest teilweise aus einem Material, welches bei Bestrahlung mit sichtbarem Licht und/oder UV-Licht keine Fluoreszenz bzw. Autofluoreszenz und/oder Absorption und/oder Reflexion und/oder Quenching-Effekte, insbesondere Fluoreszenzlöschung aufweist. Eine derartige Vorrichtung ist insbesondere mit konventionellen Licht- bzw. Fluoreszenzmikroskopen verwendbar. Der Lichteinfall kann auch durch die Elektroden hindurch erfolgen, ohne die Messergebnisse durch Fluoreszenz, Quenching, Absorption oder Reflexion des Elektrodenmaterials zu beeinflussen.

Gemäß einer Weiterbildung der erfindungsgemäßen Vorrichtung bestehen das Testsubstrat und die Elektroden zumindest teilweise aus einem biegbaren Material. Ein "biegbares Material" bezeichnet hierbei ein mechanisch flexibles Material. Die Vorrichtung eignet sich hierbei insbesondere für in-vivo Untersuchungen, da sie ihre Form an die zu untersuchenden Strukturen anpassen kann.

Gemäß einer Weiterbildung der erfindungsgemäßen Vorrichtung weisen die Elektroden jeweils einen Endbereich und einen restlichen Bereich auf. Die jeweiligen Endbereiche von mindestens zwei Elektroden sind in im Wesentlichen zueinander parallelen Reihen in dem Testbereich angeordnet. Ändert sich die Impedanz eines Endbereichs einer Elektrode, so kann darauf geschlossen werden, dass sich eine Zelle oder ein Zellteil in diesen Endbereich bewegt hat oder in diesen Endbereich hinein gewachsen ist. Dadurch kann eine Geschwindigkeit oder ein Wachstum von biologischen Substanzen in einer Richtung senkrecht zu den parallelen Reihen innerhalb des Testbereichs gemessen werden.

Gemäß einer Weiterbildung der erfindungsgemäßen Vorrichtung weisen die Elektroden jeweils einen Endbereich und einen restlichen Bereich auf. Die jeweiligen Endbereiche von mindestens zwei der Elektroden sind räumlich gleichmäßig verteilt, insbesondere in einem Array, in dem Testbereich angeordnet. Durch Messung von Impedanzänderungen der Elektroden in dem Array kann eine Bewegung oder ein Wachstum der biologischen Substanzen innerhalb des Arrays in dem Testgebiet räumlich und/oder zeitlich aufgelöst gemessen werden.

Gemäß einer Weiterbildung des erfindungsgemäßen Herstellungsverfahrens wird das Anordnen der Elektroden durch Sprühverfahren und/oder Rotationsbeschichtung und/oder Tintenstrahldruckverfahren und/oder 3D-Druck durchgeführt. Die genannten Verfahren sind einfach und kostengünstig durchzuführen und verwenden keinerlei schädliche Chemikalien, welche die zu untersuchenden biologischen Substanzen verändern könnten.

Gemäß einem weiteren Aspekt umfasst die Erfindung ein Betriebsverfahren einer Vorrichtung zum Analysieren von biologischen Substanzen in einer Testlösung. In einem ersten Verfahrensschritt wird die Testlösung auf den Testbereich des Testsubstrats aufgebracht. Anschließend werden die Impedanzen der Elektroden gemessen und die biologischen Substanzen anhand der gemessenen Impedanzen der Elektroden analysiert. Das Analysieren umfasst insbesondere das Feststellen von Zellbewegungen und/oder von zellulärem Wachstum, d.h. Ausbreitungs- und/oder Wachstumsgeschwindigkeiten und/oder Zellvitabilität.

Gemäß einem weiteren Aspekt umfasst die Erfindung ein Betriebsverfahren einer Vorrichtung zum Analysieren von biologischen Substanzen in einer Testlösung. Die Testlösung wird auf den Testbereich des Testsubstrats aufgebracht, die Impedanzen der Elektroden gemessen und eine Ausbreitungsgeschwindigkeit und/oder eine Wachstumsgeschwindigkeit der biologischen Substanzen in der Testlösung senkrecht zu den parallelen Reihen anhand der gemessenen Impedanzen der Elektroden bestimmt.

Gemäß einem weiteren Aspekt umfasst die Erfindung ein Betriebsverfahren einer Vorrichtung zum Analysieren von biologischen Substanzen in einer Testlösung. Nach dem
Aufbringen der Testlösung auf den Testbereich des Testsubstrats werden Impedanzen der rasterförmig verteilten Elektroden gemessen und eine räumliche und/oder zeitliche Verteilung der biologischen Substanzen, z.B. Zellen, in der Testlösung anhand der gemessenen Impedanzen der Elektroden bestimmt.

Die vorliegende Erfindung wird nachfolgend anhand der in den schematischen Figuren der Zeichnungen angegebenen Ausführungsbeispiele näher erläutert.

Es zeigen:
- Fig. 1: eine Vorrichtung zum Analysieren von biologischen Substanzen in einer Testlösung gemäß einer ersten Ausführungsform der vorliegenden Erfindung;
- Fig. 2: eine Vorrichtung zum Analysieren von biologischen Substanzen in einer Testlösung gemäß einer zweiten Ausführungsform der vorliegenden Erfindung;
- Fig. 3: eine Vorrichtung zum Analysieren von biologischen Substanzen in einer Testlösung gemäß einer dritten Ausführungsform der vorliegenden Erfindung;
- Fig. 4: eine Vorrichtung zum Analysieren von biologischen Substanzen in einer Testlösung gemäß einer vierten Ausführungsform der vorliegenden Erfindung;
- Fig. 5a-d: schematische Querschnittsansichten von Elektrodenstrukturen;
- Fig. 6: ein Flussdiagramm zur Erläuterung eines Herstellungsverfahrens für eine Vorrichtung zum Analysieren von biologischen Substanzen in einer Testlösung gemäß einer Ausführungsform der vorliegenden Erfindung; und
- Fig. 7-9: Flussdiagramme zur Erläuterung von Betriebsverfahren einer Vorrichtung zum Analysieren von biologischen Substanzen in einer Testlösung.

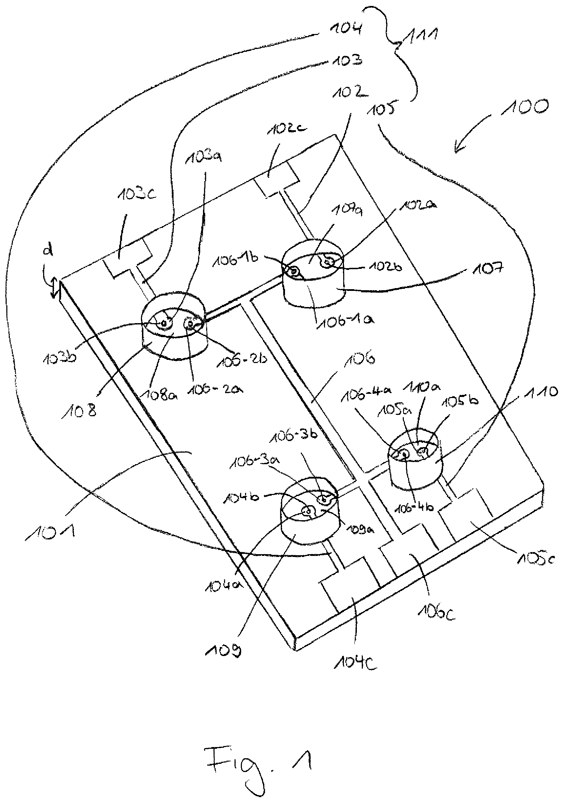

Fig. 1 zeigt eine Vorrichtung 100 zum Analysieren von biologischen Substanzen in einer Testlösung gemäß einer ersten Ausführungsform der vorliegenden Erfindung.

Die Vorrichtung 100 umfasst ein Testsubstrat 101, welches zumindest abschnittsweise aus einem transparenten Material besteht.

Das Testsubstrat 101 besteht vorzugsweise aus Kunststoff, beispielsweise PET und/oder zyklische olefinische Polymere (cyclic olefinic polymers, COP). Das Testsubstrat 101 kann jedoch auch aus anderen Materialien, insbesondere Glas und/oder Seide und/oder Papier, insbesondere auch als Folie und/oder Pflaster ausgebildet sein.

Das Testsubstrat kann insbesondere auch aus einem biegbaren Material bestehen. Eine Dicke d des Testsubstrats 101 ist hierbei klein genug gewählt, so dass sich das Testsubstrat 101 biegen lässt. Vorzugsweise besteht das Testsubstrat 101 aus einem biokompatiblen Material. Das biokompatible Material kann insbesondere ein bioinertes Material umfassen.

Auf dem Testsubstrat 101 sind erste bis vierte Fluidkammern 107, 108, 109 und 110 angeordnet. Die ersten bis vierten Fluidkammern 107 bis 110 bestehen hierbei aus kreisringförmigen Erhebungen, welche auf dem Testsubstrat 101 beispielsweise durch Kleben befestigt sind. Vorzugsweise bestehen die erste bis vierte Fluidkammern 107 bis 110 aus dem gleichen Material wie das Testsubstrat 101. Insbesondere kann das Testsubstrat 101 mit den ersten bis vierten Fluidkammern 107 bis 110 aus einem Stück gefertigt sein.

Weiter sind auf dem Testsubstrat 101 erste bis fünfte Elektroden 102, 103, 104, 105 und 106 angeordnet. Die ersten bis fünften Elektroden 102 bis 106 weisen jeweilige Kontaktierungsbereiche 102c bis 106c auf.

Die ersten bis vierten Elektroden 102 bis 105 bilden hierbei eine Elektrodengruppe 111, sind streifenförmig und einarmig ausgebildet und weisen an einem den Kontaktierungsbereichen 102c bis 105c gegenüberliegenden Ende erste bis vierte Endbereiche 102a bis 105a auf.

Die fünfte Elektrode 106 weist im Gegensatz zu der Elektrodengruppe 111 zusätzlich zu dem fünften Kontaktierungsende 106c vier Elektrodenarme mit fünften bis achten Endbereichen 106-1a, 106-2a, 106-3a und 106-4a auf.

Die ersten bis vierten Endbereiche 102a bis 105a der ersten bis vierten Elektroden 102 bis 105 und die fünften bis achten Endbereiche 106-1a bis 106-4a der fünften Elektrode 106 sind hierbei jeweils kreisförmig ausgebildet und umfassen im inneren Bereich jeweils einen kleineren kreisförmigen Innenbereich 102b, 103b, 104b, 105b, 106-1b, 106-2b, 106-3b bzw. 106-4b, welcher aus einem leitfähigen, transparenten und biokompatiblen, insbesondere bioinerten Material besteht. Dieses Material umfasst vorzugsweise Kohlenstoff-Nanoröhren und/oder leitfähige Metalloxide und/oder Polymere und/oder Hybride und/oder Metall-Nanowire. Der restliche Bereich der Elektroden, das heißt insbesondere der äußere Bereich der kreisförmigen Endbereiche 102a bis 105a bzw. 106-1a bis 106-4a, sowie der vom Endbereich verschiedene restliche Bereich der ersten bis fünften Elektroden 102 bis 106 besteht aus einem transparenten Material, welches eine höhere elektrische Leitfähigkeit als die des für die Innenbereiche verwendeten biokompatiblen Materials aufweist. Insbesondere kann das Material metallische Nanodrähte, beispielsweise Silber-Nanodrähte umfassen.

Die Erfindung ist jedoch nicht hierauf beschränkt. So können die ersten bis fünften Elektroden 102 bis 106 auch aus einem einkomponentigen transparenten Material hergestellt sein. Vorzugsweise sind die ersten bis fünften Elektroden 102 bis 106 aus einem biokompatiblen und/oder biegbaren Material ausgebildet.

Die ersten bis vierten Endbereiche 102a bis 105a der ersten bis vierten Elektroden 102 bis 105 erstrecken sich in durch die erste bis vierte Fluidkammer 107 bis 109 auf dem Testsubstrat 101 umschlossene erste bis vierte Testteilbereiche 107a, 108a, 109a und 110a. Die ersten bis vierten Testteilbereiche 107a bis 110a bilden zusammen einen Testbereich auf dem Testsubstrat 101, welcher zum Aufnehmen einer Testlösung mit biologischen Substanzen ausgebildet ist. Eine derartige Testlösung kann beispielsweise mit Hilfe einer Pipette in die durch die erste bis vierte Fluidkammer 107 bis 110 umschlossenen ersten bis vierten Testteilbereiche 107a bis 110a eingebbar sein. Die Testlösung kann auch durch mikrofluidische Zuleitungen und/oder ein Pumpensystem und/oder Automatische Einfüllvorrichtungen (Automated Liquid-Handling Systems) und/oder durch eine geeignete Oberflächenstrukturierung bzw. -funktionalisierung eingebbar sein. Weiter sind auch akustische Oberflächenwellen (surface acoustic waves, SAWs) zum Transport der Testlösung möglich.

Die biologischen Substanzen, welche beispielsweise Zellen oder Zellbestandteile umfassen, liegen hierbei vorzugsweise in Suspension in der Testlösung vor.

Die Erfindung ist nicht hierauf beschränkt. Insbesondere kann die Anzahl und Anordnung der Elektroden und Fluidkammern, sowie die Anzahl der Elektroden, welche sich jeweils in eine Fluidkammer erstrecken, variieren.

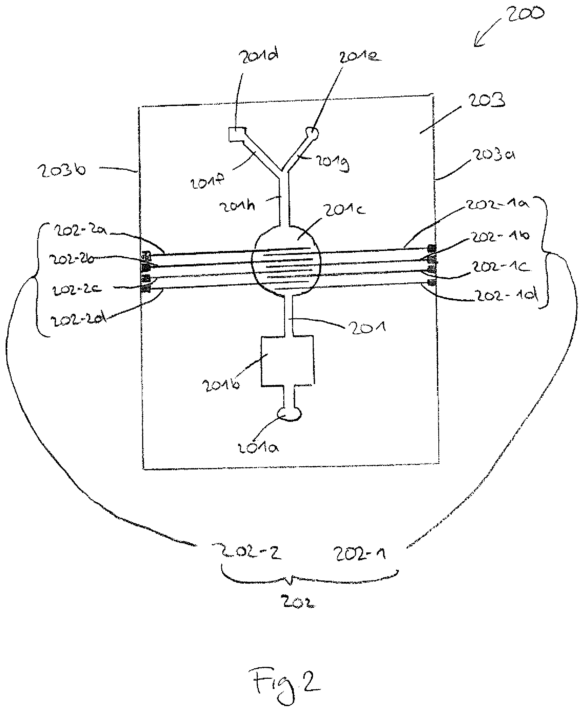

Figur 2 zeigt eine Vorrichtung 200 zum Analysieren von biologischen Substanzen in einer Testlösung gemäß einer zweiten Ausführungsform der vorliegenden Erfindung. Auf einem Testsubstrat 203, welches aus einem gleichen Material wie das Testsubstrat 101 der ersten Ausführungsform bestehen kann, das heißt insbesondere zumindest teilweise transparent ist, ist ein Mikrofluidsystem 201 angeordnet. Das Mikrofluidsystem 201 ist als Kavität ausgebildet sein und dient zum Aufnehmen einer Testlösung. Das Mikrofluidsystem 201 umfasst eine erste Fluidkammer 201d und eine zweite Fluidkammer 201e zum Einleiten von (möglicherweise verschiedenen) Testlösungen. Mittels Zuleitungen bzw. Kanälen 201f, 201g und 201h sind diese in eine dritte Fluidkammer 201c einleitbar. Das Mikrofluidsystem 201 weist eine weitere vierte Mikrofluidkammer 201b und eine fünfte Mikrofluidkammer 201a auf, welche ebenfalls durch Zuleitungen mit der dritten Mikrofluidkammer 201c verbunden sind und welche zum Ein- und/oder Ausleiten und/oder Mischen von Testlösungen bzw. Flüssigkeiten und/oder Substanzen in die dritte Mikrofluidkammer 201c ausgebildet sind.

Die Erfindung ist nicht hierauf beschränkt. So kann das Mikrofluidsystem 201 eine Vielzahl von weiteren Zuleitungen und/oder Mikrofluidkammern aufweisen, welche zum Einleiten und/oder Mischen von Testlösungen ausgebildet sind. Das Mikrofluidsystem 201 kann hierbei als Kanalsystem ausgebildet sein, wobei an den Fluidkammern Öffnungen zum Eingeben von Testlösungen angeordnet sind. Weiter können in verschiedenen Mikrofluidkammern Elektrodenstrukturen ausgebildet sein. So kann die Vorrichtung 200 weitere Elektroden umfassen, welche in die vierte Mikrofluidkammer 201 hineinragen. Weiter können die Fluidkammer beliebige Formen aufweisen.

Auf dem Testsubstrat 203 ist eine rechte Elektrodengruppe 202-1 bestehend aus ersten bis vierten Elektroden 202-1a bis 202-1d angeordnet, welche Kontaktierungsenden an einem rechten Ende 203a des Testsubstrats 203 aufweisen. Auf einer der rechten Seite 203a des Testsubstrats 203 gegenüberliegenden linken Seite 203b des Testsubstrats 203 liegen Kontaktierungsenden einer linken Elektrodengruppe 202-2 bestehend aus fünften bis achten Elektroden 202-2a bis 202-2d. Die Elektroden 202 bestehend aus der linken Elektrodengruppe 202-2 und der rechten Elektrodengruppe 202-1 sind hierbei derart auf dem Testsubstrat ausgebildet, dass Endbereiche der Elektroden 202, welche in die dritte Mikrofluidkammer 201c hineinragen, in zueinander parallelen Reihen angeordnet sind.

Die Elektroden 202 bestehen aus einem transparenten, vorzugsweise biokompatiblen und/oder biegbaren Material. Die Elektroden bestehen beispielsweise aus Kohlenstoff-Nanoröhren (CNTs) und/oder leitende Metalloxide (z.B. ZnO) und/oder Polymeren und/oder metallischen Nanodrähten, beispielsweise Silber-Nanodrähten, und/oder PEDOT-PSS und/oder Graphene und/oder Graphenoxid.

Die Erfindung ist nicht hierauf beschränkt. Insbesondere kann Anzahl der Elektroden 202 größer oder kleiner sein. Vorzugsweise ist die Anzahl der Elektroden 202 groß, um ein genaues Raster mit einer guten Ortsauflösung bereitzustellen.

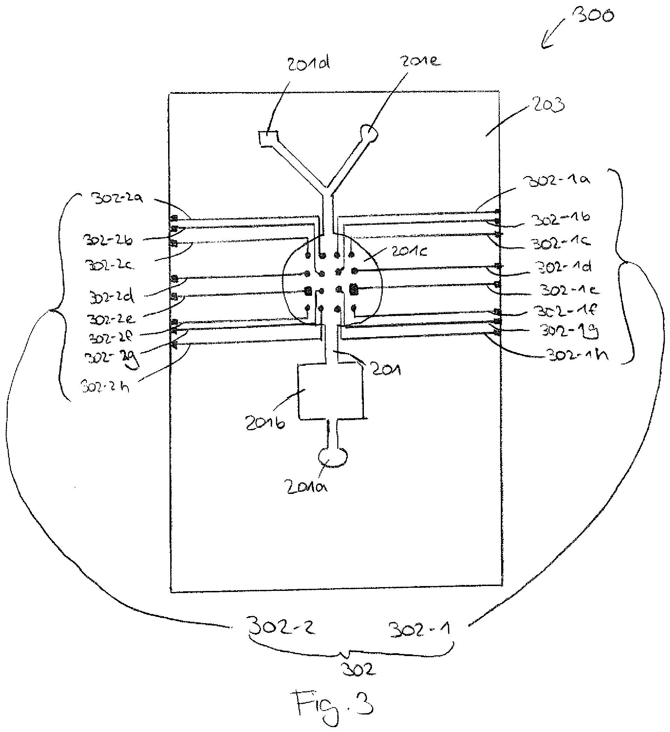

Figur 3 zeigt eine Vorrichtung 300 zum Analysieren von biologischen Substanzen in einer Testlösung gemäß einer dritten Ausführungsform der vorliegenden Erfindung. Die Vorrichtung 300 unterscheidet sich von der in Figur 2 gezeigten zweiten Ausführungsform in der Anzahl und Anordnung der Elektroden. Die Vorrichtung 300 gemäß der dritten Ausführungsform umfasst Elektroden 302, welche in eine linke Elektrodengruppe 302-2 und eine rechte Elektrodengruppe 302-1 unterteilt sind. Die rechte Elektrodengruppe 302-1 umfasst erste bis achte Elektroden 302-1a bis 302-1h und die linke Elektrodengruppe 302-2 umfasst neunte bis sechzehnte Elektroden 302-2a bis 302-2h. Die Elektrodenenden der Elektroden 302, welche in der dritten Mikrofluidkammer 201c liegen, sind punktförmig bzw. rechteckförmig (siehe Elektroden 302-2e und 302-1e) ausgebildet und in einem räumlich gleichmäßig verteilten symmetrischen 4x4-Array angeordnet. Die Elektroden bestehen wiederum aus einem der bereits oben für die zweite Ausführungsform beschriebenen Materialien. Insbesondere können die Elektroden 302 auch aus zwei verschiedenen Materialkomponenten bestehen. So können beispielsweise diejenigen Teilbereiche der Elektroden 302, welcher innerhalb der dritten Mikrofluidkammer 201c liegt, d.h. insbesondere punktförmigen bzw. rechteckförmigen Elektrodenenden der Elektroden 302, aus einem biokompatiblen Material, vorzugsweise aus Kohlenstoff-Nanoröhren und/oder aus einem Polymer ausgebildet sein, und diejenigen Teilbereiche der Elektroden, welche nicht in die dritte Mikrofluidkammer 201c hineinragen, können aus einem Material mit einer höheren elektrischen Leitfähigkeit als die des biokompatiblen Materials ausgebildet sein. Bevorzugt werden hierbei Silber-Nanodrähte verwendet.

Die Erfindung ist nicht auf die genannte Anzahl und Anordnung der Elektroden beschränkt. Insbesondere kann Anzahl der Elektroden 302 größer oder kleiner sein. Vorzugsweise ist die Anzahl der Elektroden 302 groß, um ein genaues Raster mit einer guten Ortsauflösung bereitzustellen. Weiter können alle Elektrodenenden punktförmig oder alle Elektrodenenden rechteckförmig ausgebildet sein.

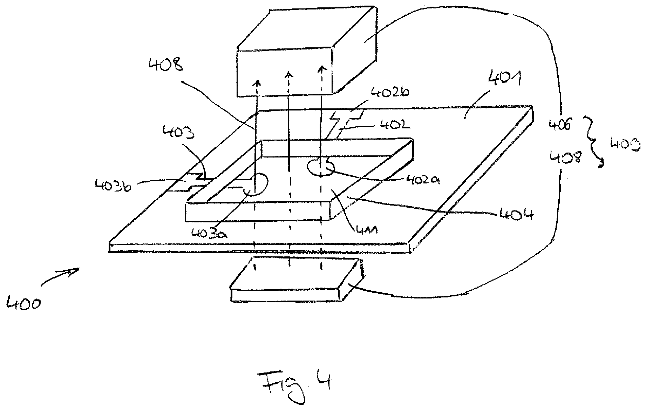

Figur 4 zeigt eine Vorrichtung 400 zum Analysieren von biologischen Substanzen in einer Testlösung gemäß einer vierten Ausführungsform der vorliegenden Erfindung. Die Vorrichtung 400 umfasst ein Testsubstrat 401, auf welchem eine Fluidkammer 404, beispielsweise ähnlich zu den in der ersten Ausführungsform in Figur 1 beschriebenen ersten bis vierten Fluidkammern 107 bis 110, ausgebildet ist, welche einen Testbereichs 411 umschließt, welcher zum Aufnehmen der Testlösung ausgebildet ist.

Auf dem Testsubstrat 401 sind eine erste Elektrode 402 und eine zweite Elektrode 403 angeordnet. Die erste Elektrode 402 und die zweite Elektrode 403 umfassen hierbei jeweils einen Kontaktierungsbereich 402b bzw. 403b sowie jeweils einen Endbereich 402a bzw. 403a, welcher jeweils innerhalb des Testbereichs 411 liegt. Die Elektroden bestehen aus einem transparenten und vorzugsweise biokompatiblen, insbesondere bioinerten Material, insbesondere einem der oben beschriebenen Materialien.

Die Vorrichtung 400 ist hierbei in ein Mikroskop 409, beispielsweise ein Fluoreszenzmikroskop eingegeben, welches eine Lichtquelle 408 und ein optisches System 406 zum Betrachten des Testbereichs 404 aufweist. Insbesondere können auch Bilder oder Videos des Testbereichs 404 über das Mikroskop 409 erfasst werden.

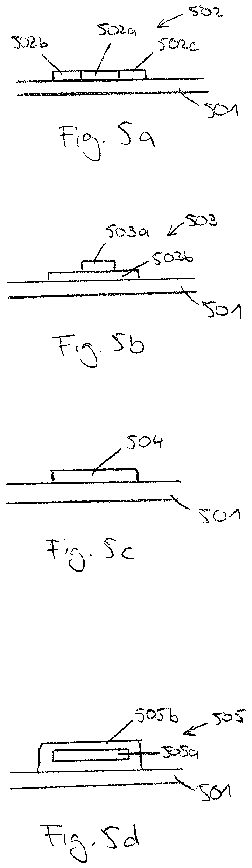

Die für die erfindungsgemäße Vorrichtung, insbesondere die in den obigen Ausführungsformen beschriebenen Vorrichtungen, verwendeten Elektroden können insbesondere eine Hybrid-Struktur aufweisen, wie in den Figuren 5a bis 5d illustriert, welche schematische Querschnittsansichten von Elektroden zeigen.

Figur 5a illustriert eine streifenförmige Elektrode 502 auf einem Testsubstrat 501. Die Elektrode 502 umfasst einen inneren streifenförmigen Abschnitt 502a aus einem vorzugsweise biokompatiblen bzw. bioinerten Material. Weiter umfasst die Elektrode 502 zwei äußere streifenförmige Abschnitte 502b und 502c, welche vorzugsweise aus Silbernanoröhren bestehen. Die gesamte Elektrode 502 ist hierbei transparent.

In Figur 5b ist eine transparente Elektrode 503 illustriert, welche einen unteren streifenförmigen Bereiche 503b umfasst, welcher auf dem Testsubstrat 501 angeordnet ist und vorzugsweise aus Silbernanoröhren besteht. Auf dem unteren streifenförmigen Bereich 503 ist ein kleinerer oberer streifenförmiger Bereich 503a angeordnet, welcher vorzugsweise aus einem biokompatiblen bzw. bioinerten Material ausgebildet ist. Der obere streifenförmige Bereich 503a kann auch aus einem nicht leitfähigen Material, insbesondere einem Polymer ausgebildet sein.

Figur 5c zeigt eine transparente Elektrode 504, wobei das Material der Elektrode 504 selbst mehrkomponentig ist, etwa sowohl Silbernanoröhren als auch Polymere aufweist.

Figur 5d zeigt eine transparente Elektrode 505, welche einen inneren leitfähigen streifenförmigen Bereich 505a und einen diesen umgebenden äußeren streifenförmigen Bereich 505b umfasst. Der äußere streifenförmige Bereich 505b besteht hierbei vorzugsweise aus einem biokompatiblen bzw. bioinerten, aber nicht zwingend leitfähigen Material, insbesondere aus Polymeren wie PDMS bzw. OSTE. Dadurch wird insbesondere eine Korrosion des inneren streifenförmigen Bereichs 505a verhindert.

Die verwendeten Elektroden können also insbesondere eine biokompatible, elektrisch isolierende Schutzschicht bzw. Passivierungsschicht aufweisen. Die in den Figuren 5a bis 5d beschriebenen Hybridstrukturen der Elektroden können hierbei auch kombiniert werden.

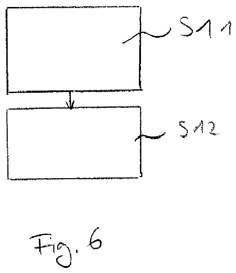

Figur 6 ist ein Flussdiagramm zur Erläuterung eines Herstellungsverfahrens für eine Vorrichtung zum Analysieren von biologischen Substanzen in einer Testlösung. In einem ersten Verfahrensschritt S11 wird ein zumindest teilweise transparentes Testsubstrat zum Aufnehmen der Testlösung ausgebildet. Das Testsubstrat wird hierbei aus einem zumindest teilweise transparenten Material, vorzugsweise aus einem Kunststoff wie PET oder COP ausgebildet. Das Testsubstrat kann auch aus anderen Materialien, insbesondere Glas und/oder Seide und/oder Papier, insbesondere auch als Folien und/oder Pflaster ausgebildet werden.

Vorzugsweise können in dem Testsubstrat Kavitäten und/oder ein Kanalsystem und/oder Fluidkammern zum Aufnehmen eines Mikrosystems ausgebildet werden. Ein Kanalsystem kann hierbei in das Substrat graviert werden. Auf dem Testsubstrat können auch Oberflächenfunktionalisierungen ausgebildet werden, insbesondere hydrophobe und/oder hydrophile Bereiche zum Transport von Flüssigkeiten. Weiter können Oberflächenstrukturierungen auf dem Testsubstrat angeordnet werden. Auf dem Testsubstrat kann auch ein Pumpensystem zum Transport von Flüssigkeiten angeordnet werden.

In einem zweiten Verfahrensschritt S12 wird eine Vielzahl von Elektroden auf dem Testsubstrat angeordnet. Hierbei ist mindestens ein Teilbereich der Elektroden aus einem transparenten Material ausgebildet. Das transparente Material umfasst insbesondere Kohlenstoff-Nanoröhren (CNTs) und/oder leitfähige Metalloxide (z.B. ZnO) und/oder Polymere und/oder metallische Nanodrähte, insbesondere Silber-Nanodrähte, und/oder PEDOT-PSS und/oder Graphene und/oder Graphenoxid. Vorzugsweise bestehen das Testsubstrat und die Elektroden zumindest teilweise aus einem biokompatiblen, insbesondere bioinerten Material. Vorzugsweise weist das Material der Elektroden keine Fluoreszenz bzw. Autofluoreszenz und/oder Absorption und/oder Reflexion und/oder Quenching-Effekte wie Fluoreszenzlöschung bei Bestrahlung mit sichtbarem Licht und/oder UV-Licht auf. Vorzugsweise ist das Material des Testsubstrats und der Elektroden biegbar.

Die Reihenfolge der Verfahrensschritte ist hierbei nicht festgelegt. So können das Ausbilden des Testsubstrats und das Anordnen der Elektroden auch gleichzeitig erfolgen.

Gemäß einer Ausführungsform kann die Vorrichtung durch 3D-Drucken in einem einzigen Verfahrensschritt hergestellt werden.

Gemäß weiteren Ausführungsformen können insbesondere flexible Testsubstrate bei niedrigen Prozesstemperaturen großflächig mit Elektrodenmaterialien beschichtet werden. Auch eine direkte Strukturierung der leitfähigen Schichten bzw. der Mikrofluidikstrukturen ist möglich.

Das Elektrodenmaterial, insbesondere Kohlenstoffnanoröhren, Silbernanowires, Graphen, Polymere oder leitende Metalloxide wie Zinkoxid, wird hierzu vorzugsweise in Lösung gebracht, beispielsweise mit Hilfe von Tensiden. Die Lösung mit dem Elektrodenmaterial wird dann auf das Testsubstrat zum Anordnen der Elektroden aufgebracht.

Das Anordnen der Elektroden kann hierbei gemäß einer Ausführungsform mit Hilfe eines Tintenstrahldruckers durchgeführt werden. Das Elektrodenmaterial wird hierbei auf das Testsubstrat aufgedruckt und vorzugsweise strukturiert.

Gemäß einer weiteren Ausführungsform können die Elektroden durch ein Sprühverfahren ausgebildet werden. Hierzu wird zur Strukturierung zuerst eine Schattenmaske auf das Testsubstrat aufgebracht, anschließend das Elektrodenmaterial in Lösung aufgesprüht und schließlich die Schattenmaske wieder entfernt. Die Schattenmaske kann beispielsweise mittels Laser-Cutting hergestellt werden. Optional kann anschließend ein Reinigungsschritt stattfinden, welcher insbesondere Tenside und andere nichtbiokompatible Materialien entfernt. Des Weiteren kann das Verfahren einen optionalen Sinterschritt umfassen, beispielsweise zur Erhöhung der elektrischen Leitfähigkeit.

Gemäß einer weiteren Ausführungsform können die Elektroden durch Rotationsbeschichtung auf dem Testsubstrat angeordnet werden.

Die genannten Herstellungsverfahren können auch beliebig kombiniert werden.

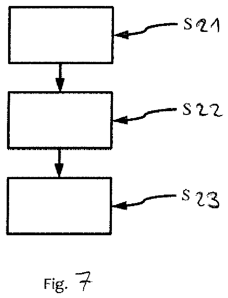

Figur 7 zeigt ein Flussdiagramm zur Erläuterung eines Betriebsverfahrens einer Vorrichtung zum Analysieren von biologischen Substanzen in einer Testlösung. Die Vorrichtung kann hierbei insbesondere eine der in den oben genannten Ausführungsbeispielen beschriebenen Vorrichtungen sein. In einem ersten Schritt S21 wird die Testlösung auf den Testbereich des Testsubstrats aufgebracht. Das Aufbringen kann beispielsweise mittels einer Pipette oder durch geeignete Öffnungen oder durch ein Kanalsystem erfolgen. Die biologischen Substanzen, beispielsweise Zellen oder Zellbestandteile, liegen hierbei in Suspension in der Testlösung, insbesondere einem Mikrofluid vor.

In einem zweiten Schritt werden Impedanzen der Elektroden gemessen. Hierbei wird eine Wechselspannung zwischen verschiedenen Elektroden angelegt, so dass in dem Testbereich ein elektrisches Feld zwischen den Elektroden aufgebaut wird. Ändert sich das elektrische Feld aufgrund einer Änderung der biologischen Substanzen zwischen den beiden Elektroden, so wird auch eine frequenzabhängige Änderung der Impedanzen der Elektroden gemessen.

Anhand der gemessenen Impedanzen der Elektroden werden dann in einem dritten Verfahrensschritt S23 die biologischen Substanzen analysiert.

Ändert sich beispielsweise die Impedanz an einer Elektrode, so kann darauf geschlossen werden, dass sich eine biologische Substanz im Bereich der Elektrode bewegt. Dadurch kann auf ein Wachstum der biologischen Substanz, die Zellvitabilität oder eine Bewegung der biologischen Substanz rückgeschlossen werden.

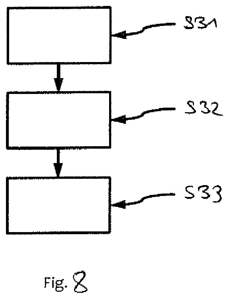

Figur 8 zeigt ein Flussdiagramm zur Erläuterung eines Betriebsverfahrens einer Vorrichtung zum Analysieren von biologischen Substanzen in einer Testlösung. Die Vorrichtung weist hierbei eine Vielzahl von Elektroden auf, welche jeweils einen Endbereich und einen restlichen Bereich aufweisen, wobei die jeweiligen Endbereiche der Vielzahl von Elektroden im Wesentlichen parallel zueinander ausgebildet sind. Die Vorrichtung kann also insbesondere eine Vorrichtung 200 gemäß der zweiten Ausführungsform sein, wie in Figur 2 gezeigt.

Parallel zum oben beschriebenen Betriebsverfahren werden in einem ersten Schritt S31 eine Testlösung auf das Testsubstrat aufgebracht und in einem zweiten Schritt S32 Impedanzen der Elektroden gemessen.

In einem dritten Schritt S33 werden eine Ausbreitungsgeschwindigkeit und/oder ein Wachstum, insbesondere eine Wachstumsgeschwindigkeit, der biologischen Substanzen in der Testlösung senkrecht zu den parallelen Reihen anhand der gemessenen Impedanzen der Elektroden bestimmt. Ändert sich beispielsweise die Impedanz einer Elektrode, so kann darauf geschlossen werden, dass sich die biologische Substanz an dieser Elektrode befindet. Durch Messen der Zeitspanne zwischen einem ersten Zeitpunkt, an welchem sich die biologische Substanz an einer ersten Elektrode befindet und einem zweiten Zeitpunkt, an welchem sich die biologische Substanz an einer benachbarten zweiten Elektrode befindet, kann anhand des bekannten Abstandes zwischen zwei benachbarten Elektroden die Ausbreitungsgeschwindigkeit und/oder das Wachstum, das heißt die Wachstumsgeschwindigkeit, der biologischen Substanz senkrecht zu den Elektroden berechnet werden.

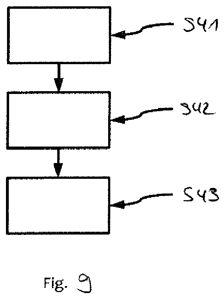

Figur 9 zeigt ein Flussdiagramm zur Erläuterung eines Betriebsverfahrens einer Vorrichtung zum Analysieren von biologischen Substanzen in einer Testlösung. Analog zu den oben beschriebenen Betriebsverfahren wird in einem ersten Schritt S41 die Testlösung auf dem Testbereich des Testsubstrats aufgebracht und in einem zweiten Schritt S42 werden Impedanzen der Elektroden gemessen. Die Elektroden umfassen hierbei jeweils einen Endbereich und einen restlichen Bereich, wobei die jeweiligen Endbereiche der Elektroden räumlich gleichmäßig verteilt, das heißt insbesondere in einem Array in dem Testbereich des Testsubstrats angeordnet sind. Insbesondere ist das Betriebsverfahren für eine Vorrichtung 300 gemäß der dritten Ausführungsform, wie in Figur 3 erläutert, geeignet.

Hierbei wird in einem dritten Schritt S43 eine räumliche und/oder zeitliche Verteilung der biologischen Substanzen in der Testlösung anhand der gemessenen Impedanz der Elektroden bestimmt. Hierbei wird bei Änderung der Impedanz einer Elektrode rückgeschlossen, dass die biologischen Substanzen zum Zeitpunkt der Änderung der Impedanz die den Endbereich der Elektrode erreichen, das heißt berührt. Durch Messen der Zeitspanne zwischen einem ersten Zeitpunkt, an welchem sich die biologische Substanz an einem Endbereich einer ersten Elektrode befindet und einem zweiten Zeitpunkt, an welchem sich die biologische Substanz an einem Endbereich einer benachbarten zweiten Elektrode befindet, kann anhand des bekannten Abstandes zwischen den zwei Endbereichen der benachbarten Elektroden die Ausbreitungsgeschwindigkeit und die räumliche und/oder zeitliche Verteilung der biologischen Substanz in der Testlösung berechnet werden.

Die genannten Ausführungsformen können auch kombiniert werden. So kann eine Vorrichtung gemäß einer Ausführungsform sowohl Elektroden zum Bestimmen einer räumlichen und/oder zeitlichen Verteilung der biologischen Substanzen umfassen, etwa in einer Anordnung wie in Figur 3 illustriert, als auch Elektroden zum Bestimmen einer Ausbreitungsgeschwindigkeit und/oder eines Wachstums der biologischen Substanzen, etwa in einer Anordnung wie in Figur 2 illustriert.

## Patentansprüche

1. Vorrichtung (100; 200; 300; 400) zum Analysieren von biologischen Substanzen in einer Testlösung, mit
einem zumindest teilweise transparenten Testsubstrat (101; 203; 303; 401) mit einem Testbereich (107a, 108a, 109a, 110a; 211; 411) zum Aufnehmen der Testlösung; und einer Vielzahl von auf dem Testsubstrat (101; 203; 303; 401) angeordneten und sich in den Testbereich (107a, 108a, 109a, 110a; 211; 411) erstreckenden Elektroden (111, 106; 201, 202; 301, 302; 402, 403);
wobei jeweils zumindest ein Teilbereich der Elektroden (111, 106; 201, 202; 301, 302; 402, 403) im Testbereich (107a, 108a, 109a, 110a; 211; 411) aus einem transparenten Material ausgebildet ist.

2. Vorrichtung nach Anspruch 1, wobei das Testsubstrat (101; 203; 303; 401) eine Kavität zum Aufnehmen der Testlösung aufweist.

3. Vorrichtung (100; 200; 300; 400) nach einem der Ansprüche 1 oder 2, wobei das transparente Material aus welchem zumindest der Teilbereich der Elektroden (111, 106; 201, 202; 301, 302; 402, 403) ausgebildet ist, Kohlenstoff-Nanoröhren und/oder leitenden Oxide und/oder Polymere und/oder metallische Nanodrähte, insbesondere Silber-Nanodrähte, und/oder PEDOT-PSS und/oder Graphen und/oder Graphenoxid umfasst.

4. Vorrichtung (100; 200; 300; 400) nach einem der Ansprüche 1 bis 3, wobei das Testsubstrat (101; 203; 303; 401) und die Elektroden (111, 106; 201, 202; 301, 302; 402, 403) zumindest teilweise aus einem biokompatiblen, insbesondere bioinerten Material bestehen.

5. Vorrichtung (100; 200; 300; 400) nach Anspruch 4,
wobei die Elektroden (111, 106; 201, 202; 301, 302; 402, 403) jeweils einen Endbereich und einen restlichen Bereich aufweisen; und
wobei die Endbereiche der Elektroden (111, 106; 201, 202; 301, 302; 402, 403) zumindest teilweise aus einem biokompatiblen, insbesondere bioinerten Material besteht, und
wobei die restlichen Bereiche der Elektroden (111, 106; 201, 202; 301, 302; 402, 403) aus einem Material besteht, welches eine höhere elektrische Leitfähigkeit als die des biokompatiblen Materials aufweist.

6. Vorrichtung (100; 200; 300; 400) nach einem der Ansprüche 1 bis 5, wobei die Elektroden (111, 106; 201, 202; 301, 302; 402, 403) zumindest teilweise aus einem Material bestehen, welches bei Bestrahlung mit sichtbarem Licht und/oder UV-Licht keine Fluoreszenz und/oder Absorption und/oder Reflexion und/oder Quenching-Effekte aufweist.

7. Vorrichtung (100; 200; 300; 400) nach einem der Ansprüche 1 bis 6, wobei das Testsubstrat (101; 203; 303; 401) und die Elektroden (111, 106; 201, 202; 301, 302; 402, 403) zumindest teilweise aus einem biegbaren Material bestehen.

8. Vorrichtung (200) nach einem der Ansprüche 1 bis 7,
wobei die Elektroden (111, 106; 201, 202; 301, 302; 402, 403) jeweils einen Endbereich und einen restlichen Bereich aufweisen; und
wobei die Endbereichen von mindestens zwei Elektroden (202) in im Wesentlichen zueinander parallelen Reihen in dem Testbereich (107a, 108a, 109a, 110a; 211; 411) angeordnet sind.

9. Vorrichtung (300) nach einem der Ansprüche 1 bis 8,
wobei die Elektroden (111, 106; 201, 202; 301, 302; 402, 403) jeweils einen Endbereich und einen restlichen Bereich aufweisen; und
wobei die jeweiligen Endbereiche von mindestens zwei der Elektroden (302) räumlich gleichmäßig verteilt, insbesondere in einem Array, in dem Testbereich (107a, 108a, 109a, 110a; 211; 411) angeordnet sind.

10. Herstellungsverfahren für eine Vorrichtung (100; 200; 300; 400) zum Analysieren von biologischen Substanzen in einer Testlösung mit den Schritten:
Ausbilden (S11) eines zumindest teilweise transparenten Testsubstrats (101; 203; 303; 401) mit einem Testbereich (107a, 108a, 109a, 110a; 211; 411) zum Aufnehmen der Testlösung; und
Anordnen (S12) von einer Vielzahl von Elektroden (111, 106; 201, 202; 301, 302; 402, 403) auf dem Testsubstrat;
wobei jeweils zumindest ein Teilbereich der Elektroden (111, 106; 201, 202; 301, 302; 402, 403) im Testbereich (107a, 108a, 109a, 110a; 211; 411) aus einem transparenten Material ausgebildet wird.

11. Herstellungsverfahren nach Anspruch 11, wobei das Anordnen der Elektroden (111, 106; 201, 202; 301, 302; 402, 403) durch Sprühverfahren und/oder Rotationsbeschichtung und/oder Tintenstrahldruckverfahren und/oder 3D-Druck durchgeführt wird.

12. Betriebsverfahren einer Vorrichtung (100; 200; 300; 400) nach einem der Ansprüche 1 bis 10, mit den Schritten
Aufbringen (S21) der Testlösung auf den Testbereich (107a, 108a, 109a, 110a; 211; 411) des Testsubstrats (101; 203; 303; 401),
Messen (S22) von Impedanzen der Elektroden (111, 106; 201, 202; 301, 302; 402, 403), und
Analysieren (S23) der biologischen Substanzen anhand der gemessenen Impedanzen der Elektroden (111, 106; 201, 202; 301, 302; 402, 403).

13. Betriebsverfahren einer Vorrichtung (100; 200; 300; 400) nach Anspruch 8, mit den Schritten
Aufbringen (S31) der Testlösung auf den Testbereich (107a, 108a, 109a, 110a; 211; 411) des Testsubstrats (101; 203; 303; 401),
Messen (S32) von Impedanzen der Elektroden (111, 106; 201, 202; 301, 302; 402, 403),
Bestimmen (S33) einer Ausbreitungsgeschwindigkeit und/oder eines Wachstums der biologischen Substanzen in der Testlösung senkrecht zu den parallelen Reihen anhand der gemessenen Impedanzen der Elektroden (111, 106; 201, 202; 301, 302; 402, 403).

14. Betriebsverfahren einer Vorrichtung (100; 200; 300; 400) nach Anspruch 9, mit den Schritten
Aufbringen (S41) der Testlösung auf den Testbereich (107a, 108a, 109a, 110a; 211; 411) des Testsubstrats (101; 203; 303; 401),
Messen (S42) von Impedanzen der Elektroden (111, 106; 201, 202; 301, 302; 402, 403),
Bestimmen (S43) einer räumlichen und/oder zeitlichen Verteilung der biologischen Substanzen in der Testlösung anhand der gemessenen Impedanzen der Elektroden (111, 106; 201, 202; 301, 302; 402, 403).
